# EUROPÄISCHE PATENTSCHRIFT

(11) **EP 1 839 061 B1**
(45) Veröffentlichungstag und Bekanntmachung des Hinweises auf die Patenterteilung: **12.03.2014**
(21) Anmeldenummer: 06706177.0
(22) Anmeldetag: 09.01.2006
(51) Int. Cl.: C12N 7/02, B01D 15/00

(54) **VERFAHREN ZUR UNSPEZIFISCHEN SEPARATION VON ZELLEN AUS FLUESSIGEN MEDIEN UND DESSEN VERWENDUNG**
METHOD FOR THE UNSPECIFIC SEPARATION OF CELLS FROM LIQUID MEDIA AND USE THEREOF
PROCEDE DE SEPARATION NON SPECIFIQUE DE CELLULES DE MILIEUX LIQUIDES ET UTILISATION DUDIT PROCEDE

(30) Priorität: 18.01.2005 DE 102005002343
(43) Veröffentlichungstag der Anmeldung: 03.10.2007
(73) Patentinhaber: Fraunhofer-Gesellschaft zur Förderung der angewandten Forschung e.V., 80686 München (DE); Rheinische Friedrich-Wilhelms-Universität Bonn, 53113 Bonn (DE)
(72) Erfinder: HOLLÄNDER, Andreas, 14482 Potsdam (DE); KEUSGEN, Michael, 53359 Rheinbach (DE); KRÄMER, Johannes, 53913 Swisttal (DE); FERNER, Ansgar, 56410 Montabaur (DE)
(74) Vertreter: Pfenning, Meinig & Partner GbR
(86) Internationale Anmeldenummer: PCT/EP2006/000105
(87) Internationale Veröffentlichungsnummer: WO 2006/077020

(56) Entgegenhaltungen:
- DE-C2- 4 042 579
- US-A- 3 970 518
- US-A- 4 415 665
- US-A- 4 927 749
- US-A- 5 185 415
- US-A1- 2001 009 757
- US-A1- 2001 009 759
- US-A1- 2002 117 453
- PATENT ABSTRACTS OF JAPAN Bd. 2002, Nr. 10, 10. Oktober 2002 (2002-10-10) & JP 2002 165591 A (JSR CORP), 11. Juni 2002 (2002-06-11)

## Beschreibung

Die Erfindung betrifft ein Verfahren zur unspezifischen Separation von Zellen aus flüssigen Medien, das auf einer Adsorption der der Spezies an einem entsprechend funktionalisierten Trägermaterial basiert. Verwendung findet das erfindungsgemäße Verfahren zur Abtrennung von Zellen.

Die Abtrennung und Anreicherung von Mikroorganismen ist ein großes Problem in der mikrobiellen Diagnostik. Hier stehen zeitaufwendige Filtrationsmethoden (WO 01/52968) oder Zentrifugationstechniken (JP 3270701) zur Verfügung. Diese versagen aber, wenn die zu untersuchenden Lösungen viskos sind oder Festkörper enthalten. Andere Verfahren basieren auf Immunocapturing, wobei Festkörper, die mit Antikörpern belegt sind, zum Fangen der Zellen eingesetzt werden. Die Festkörper können magnetische Partikel sein (z.B. CA 1127571, AU 533747). Diese Techniken sind durch den Einsatz von Antikörpern recht teuer. Ebenfalls ist die Bindung der Mikroorganismen stark von äußeren Einflüssen abhängig, wie z.B. von der Temperatur, der Fließgeschwindigkeit und den dadurch auftretenden Scherkräften.

Bei der Reinigung von Viren ist Anwendung der Hochdruck-Flüssigchromatographie mit Ionenaustauscherharzen beschrieben (WO 00/40702).

Die Analyse von Zellen, insbesondere von tierischen Zellen, wird heute mittels Flow-Cytometrie durchgeführt (US 6793642, US 2004189977, DE 199 45 553). Dabei werden die Zellen in einer Kapillare vereinzelt und auf Grund eines Signals, z.B. Lichtstreuung, Fluoreszenz o.ä., sortiert. Das ist ein langwieriger und wenig selektiver Prozess.

Ausgehend hiervon war es Aufgabe der vorliegenden Erfindung, ein Verfahren bereitzustellen, dass die Separation und Isolierung von Zellen auf einfache Weise und damit kostengünstig ermöglicht.

Diese Aufgabe wird durch das Verfahren mit den Merkmalen des Anspruchs 1 gelöst. In Anspruch 12 wird die Verwendung des erfindungsgemäßen Verfahrens beschrieben. Die weiteren abhängigen Ansprüche zeigen vorteilhafte Weiterbildungen auf.

Erfindungsgemäß wird ein Verfahren zur unspezifischen Separation von Zellen aus flüssigen Medien bereitgestellt, bei dem
a) ein Trägermaterial, ausgewählt aus der Gruppe bestehend aus Polymeren, Keramiken und Gläsern, aktiviert wird, wobei die Aktivierung durch eine oxidative Aktivierung mit einem Oxidationsmittel, durch eine photochemische Aktivierung oder durch Plasma-Behandlung erfolgt, an der Oberfläche chemisch hydrophil funktionalisiert wird, um eine Chemisorption der Zellen zu ermöglichen, wobei die Chemisorption auf einer ionischen Wechselwirkung beruht und die funktionellen Gruppen ausgewählt sind aus der Gruppe bestehend aus Ammonium-, Carboxyl-, Carboxylat-, Sulphonsäure-, Sulphonat- und Phosphatgruppen, und/oder aufgrund kovalenter Wechselwirkungen, wobei die funktionellen Gruppen ausgewählt sind aus der Gruppe bestehend aus Aktivester, Säureamiden, Halogeniden und Säurehalogeniden,
b) das Trägermaterial mit dem flüssigen Medium in Kontakt gebracht wird und die Zellen am Trägermaterial adsorbiert werden,
c) das mit den Zellen beladene Trägermaterial von flüssigem Medium abgetrennt wirdund
d) zumindest ein Teil der Zellen durch Elution des beladenen Trägermaterials mit einer Flüssigkeit wiedergewonnen und die wiedergewonnenen Zellen zur qualitativen Bestimmung analysiert werden.

Zahlreiche Mikroorganismen zeigen eine feste oder reversible Bindung zu ionischen Oberflächen. Dabei ist die Bindung abhängig von der jeweiligen Mikroorganismusspezies. Diese Interaktionen sind jedoch nicht gleich stark, sondern sind von Spezies zu Spezies verschieden. Aufgrund dieser qualitativ und quantitativ unterschiedlichen Wechselwirkung ist eine Separierung und Auftrennung unterschiedlicher Zellen möglich, z.B. das Trennen unterschiedlicher bakterieller Spezies.

Die Wechselwirkung von Zellen mit den Oberflächen der Trägerstruktur kann durch chemische Funktionalisierung der Oberfläche beeinflusst werden, wobei weiterhin die Wechselwirkung zwischen den Zellen und dem Trägermaterial durch die Wahl des Puffers über z.B. die Ionenstärke und den pH-Wert modulierbar ist. Die Bindung kann dabei sehr fest und nicht lösbar eingestellt werden. Auf der anderen Seite ist es ebenso möglich, dass die Bindung reversibel ist.

Erfindungsgemäß wird das Trägermaterial ausgewählt aus der Gruppe bestehend aus Polymeren, Keramiken und Gläsern. Das Trägermaterial kann dabei in Form eines Pulvers, eines Granulats, einer Folie, einer Membran, eines Sinterkörpers oder eines Schaums eingesetzt werden.

Erfindungsgemäß wird das Trägermaterial hydrophil funktionalisiert. Dabei ist es möglich, dass eine direkte Anbindung der funktionellen Gruppen an dem Trägermaterial erfolgt. Eine weitere Variante sieht vor, dass die funktionellen Gruppen über einen Spacer an das Trägermaterial gebunden werden.

Erfindungsgemäß handelt es sich bei den Adsorptionskräften, die zwischen Trägermaterial und Zellen wirken, um chemisorptive Wechselwirkungen.

Ein bevorzugter Bereich der Chemisorption beruht dabei auf ionischen Wechselwirkungen. In diesem Falle sind die funktionellen Gruppen ausgewählt aus der Gruppe bestehend aus Ammonium-, Carboxyl-, Carboxylat-, Sulfonsäure-, Sulfonat-, Phosphatgruppen und Proteine.

Eine andere bevorzugte Variante sieht vor, dass die Chemisorption auf einer kovalenten Wechselwirkung beruht. In diesem Falle sind die funktionellen Gruppen ausgewählt aus der Gruppe bestehend aus Aktivester, Säureamide, Epoxide, Halogenide, Säurehalogenide und C-C-Mehrfachbindungen.

Hinsichtlich der Ladung des funktionalisierten Trägermaterials erfolgt die Anpassung ebenfalls hinsichtlich der zu separierenden bzw. isolierenden Spezies. Somit kann das Trägermaterial neutralen, kationischen oder anionischen Charakter besitzen.

Die zu isolierenden Zellen sind vorzugsweise ausgewählt aus der Gruppe der prokaryotischen und der eukaryotischen Zellen bzw. aus der Gruppe der ArchaeBakterien. Besonders bevorzugt sind die Zellen ausgewählt aus der Gruppe bestehend aus Bakterien, Pilzen, Algen, Sporen, pflanzlichen Zellen, tierischen Zellen, Zellen aus Zellkulturen und gentechnologisch veränderten Zellen.

Die Verfahrensführung hinsichtlich des Inkontaktbringens der Zellen mit dem Trägermaterial ist nicht beschränkend ausgelegt und umfasst alle aus dem Stand der Technik bekannten Methoden hierzu. Eine Variante sieht beispielsweise vor, dass das Trägermaterial in dem flüssigen Medium dispergiert wird. Eine andere bevorzugte Variante sieht vor, dass das Trägermaterial von dem flüssigen Medium umströmt wird. Eine dritte bevorzugte Variante sieht vor, dass das Trägermaterial in Form eines porösen Körpers vorliegt, durch dessen Poren dann das flüssige Medium hindurchströmen kann. All diese Varianten ermöglichen eine Adsorption der zu isolierenden Spezies, sofern die Wechselwirkungen zwischen Trägermaterial und Zellen stark genug sind.

Erfindungsgemäß wird das Trägermaterial vor der Funktionalisierung zusätzlich aktiviertDie Aktivierung kann dabei chemisch erfolgen. Eine bevorzugte Variante ist eine oxidative Aktivierung mit Oxidationsmittel, wie z.B. H₂O₂, Peroxiden, Chromschwefelsäure, Iod- und Bromverbindungen. Ebenso ist eine photochemische Aktivierung durch UV-Licht oder auch Mikrowellenstrahlung bevorzugt.

Die chemische Funktionalisierung erfolgt dann durch entsprechend weitere Reaktionen, die auch mehrstufig geführt werden können, mit gasförmigen Reagenzien, z.B. Diamin oder auch mit Flüssigkeiten, z.B. Lösungen von Polyethylenimin oder Polyethylenglycol.

Eine andere Variante der Aktivierung betrifft die physikalische Aktivierung, z.B. mittels eines Plasmas, d.h. einer elektrischen Entladung.

Die Abtrennung des Trägermaterials vom flüssigen Medium unterliegt keinerlei Beschränkung, so dass auch hier alle aus dem Stand der Technik bekannten varianten eingesetzt werden können. Vorzugsweise zählen zu diesen die Filtration, die Sedimentation oder die Zentrifugation.

Erfindungsgemäß wird zumindest ein Teil der Zellen durch Elution des beladenen Trägermaterials mit einer Flüssigkeit wiedergewonnen. Hierbei handelt es sich somit dann um eine Isolierung der einzelnen Mikroorganismenspezies und nicht allein um eine quantitative Separation.

Die wiedergewonnenen Zellen werden erfindungsgemäß im Anschluss analysiert. Dies ermöglicht nicht alleine eine quantitative Abtrennung von Zellen aus Flüssigkeiten, sondern auch gleichzeitig eine qualitative Bestimmung, welche Arten von Zellen in der entsprechenden Flüssigkeit enthalten gewesen sind.

Bei der reversiblen Bindung von Zellen an das Trägermaterial können bindungskinetische Differenzen von verschiedenen Zellen mit der gegebenen Oberfläche für die Separation benutzt werden. Die Elution erfolgt durch einen Puffer mit entsprechender Ionenstärke und entsprechend eingestelltem pH-Wert. Die einzelnen Spezies zeigen in diesem Fall ein unterschiedliches Retentionsverhalten gegenüber dem Trägermaterial.

Verwendung findet das erfindungsgemäße Verfahren zur Abtrennung von Zellen aus Flüssigkeiten. Zu diesen zählen insbesondere Wasser, flüssige Lebensmittel, Flüssigkeiten aus Lebensmitteln, Körperflüssigkeiten oder Ausscheidungen.

Anhand der nachfolgenden Figuren und Beispiele soll der erfindungsgemäße Gegenstand näher erläutert werden, ohne diesen auf die hier gezeigte Ausführungsbreite beschränken zu wollen.
- Fig. 1: zeigt ein Diagramm, das die Wiederfindungsrate bestimmter Bakterientypen in einem flüssigen Medium zeigt, bei dem das erfindungsgemäße Verfahren mit einem unbehandelten Trägermaterial aus Polyethylen durchgeführt wurde.
- Fig. 2: zeigt ein Diagramm, das die Wiederfindungsrate in einem flüssigen Medium darstellt, für das das erfindungsgemäße Verfahren mit einem chemisch funktionalisierten Trägermaterial (aminiert) durchgeführt wurde.
- Fig. 3: zeigt ein Diagramm, das die Wiederfindungsrate in einem flüssigen Medium darstellt, für das das erfindungsgemäße Verfahren mit einem chemisch funktionalisierten Trägermaterial (carboxyliert) durchgeführt wurde.

### Beispiel 1

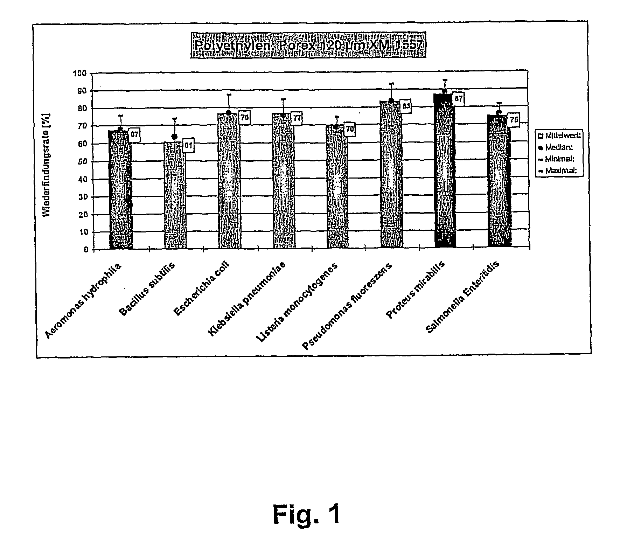

Über zylindrische Formkörper aus gesintertem Polyethylenpulver mit 5 mm Durchmesser und 5 mm (ohne weitere Funktionalisierung) Höhe werden Suspensionen von 1,6 10⁶ KbE/g mit einer Geschwindigkeit vom 1 ml/min gegeben. Die Auszählung der Zellen im Eluat ergab die in Fig. 1 dargestellten Werte.

### Beispiel 2

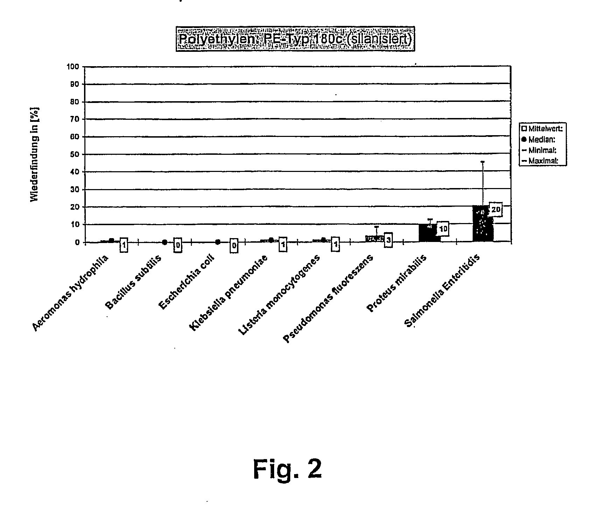

Zylindrische Formkörper aus gesintertem Polyethylenpulver mit 5 mm Durchmesser und 5 mm Höhe werden in einem Niederdruckplasma an den Oberflächen oxidiert und anschließend mit Aminopropyltriethoxysilan umgesetzt. Über diese funktionalisierten Sinterkörper werden Suspensionen von 1,6 10⁶ KbE/g mit einer Geschwindigkeit vom 1 ml/min gegeben. Die Auszählung der Zellen im Eluat ergab die in Fig. 2 dargestellten Werte.

### Beispiel 3

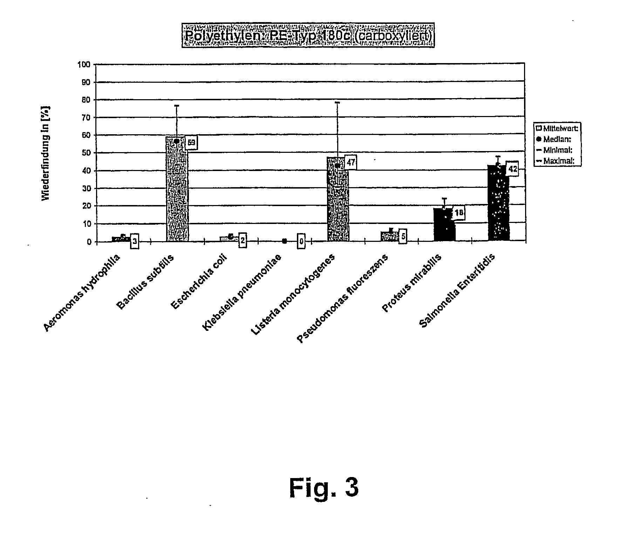

Zylindrische Formkörper aus gesintertem Polyethylenpulver mit 5 mm Durchmesser und 5 mm Höhe werden in einem Niederdruckplasma an den Oberflächen oxidiert und anschließend mit Aminopropyltriethoxysilan umgesetzt. An die Aminogruppen werden über die Reaktion mit Bernsteinsäureanhydrid Carboxylgruppen gebunden. Über diese funktionalisierten Sinterkörper werden Suspensionen von 1,6 10⁶ KbE/g mit einer Geschwindigkeit vom 1 ml/min gegeben. Die Gram-positiven Zellen von Bacillus subtilis, Lysteria monocytogenes und Salmonella Enteritidis werden im Eluat angereichert. Die Auszählung der Zellen im Eluat ergab die in Fig. 3 dargestellten Werte.

### Beispiel 4

Ein Polypropylenpulver wird an der Oberfläche mit kovalent gebundenem Polyethylenimin versehen. 0,1 g des Pulvers werden in 1 1 einer Suspension von 10⁴ Zellen je ml von *E. coli* dispergiert. Nach 1 Stunde wird das Pulver mit allen zuvor dispergierten Zellen an der Oberfläche gebunden abfiltriert. Die auf der Pulveroberfläche angereicherten Zellen werden nun der molekularbiologischen Analyse (z.B. mit PCR) unterzogen.

### Beispiel 5

Ein poröser Körper von 50 mm Länge und einem Durchmesser von 5 mm wird fest in eine druckfeste Kartusche eingepasst und in eine Durchfluss-Anlage eingebracht. Anschließend wird an einem Ende ("Eintrittsende") eine Bakterienprobe aufgebracht. Diese wird anschließend mit einer wässrigen Lösung, deren Ionenstärke und pH variabel ist, über den Polymersinterkörper geleitet. Dabei tritt das Zellmaterial in Wechselwirkung mit dem Polymersinterkörper und wird in unterschiedlicher Wiese reterniert. Dadurch können Mischungen unterschiedlicher Bakterien getrennt und am anderen Ende des Polymersinterkörpers ("Austritts-ende") mit einem geeigneten Detektor (Lichtstreuung, UV, Leitfähigkeit, Fluoreszenz, etc.) nachgewiesen werden. Einzelne Bakterienfraktionen können aufgefangen werden und anschließend einer nachgeschalteten Identifizierung durch immunologische und molekularbiologische Methoden (z.B. PCR) identifiziert und charakterisiert werden.

## Patentansprüche

1. Verfahren zur unspezifischen Separation und Analyse von Zellen aus flüssigen Medien, bei dem
a) ein Trägermaterial, ausgewählt aus der Gruppe bestehend aus Polymeren, Keramiken und Gläsern, aktiviert wird, wobei die Aktivierung durch eine oxidative Aktivierung mit einem Oxidationsmittel, durch eine photochemische Aktivierung oder durch Plasma-Behandlung erfolgt, und dann an der Oberfläche chemisch hydrophil funktionalisiert wird, um eine Chemisorption der Zellen zu ermöglichen, wobei die Chemisorption auf einer ionischen Wechselwirkung beruht und die funktionellen Gruppen ausgewählt sind aus der Gruppe bestehend aus Ammonium-, Carboxyl-, Carboxylat-, Sulphonsäure-" Sulphonat- und Phosphatgruppen, und/oder aufgrund kovalenter Wechselwirkungen, wobei die funktionellen Gruppen ausgewählt sind aus der Gruppe bestehend aus Aktivester, Säureamiden, Halogeniden und Säurehalogeniden,
b) das Trägermaterial mit dem flüssigen Medium in Kontakt gebracht wird und die Zellen am Trägermaterial adsorbiert werden,
c) das mit den Zellen beladene Trägermaterial vom flüssigen Medium abgetrennt wird und
d) zumindest ein Teil der Zellen durch Elution des beladenen Trägermaterials mit einer Flüssigkeit wiedergewonnen und die wiedergewonnenen Zellen zur qualitativen Bestimmung analysiert werden.

2. Verfahren nach dem vorhergehenden Anspruch, **dadurch gekennzeichnet, dass** das Trägermaterial in Form eines Pulvers, eines Granulats, einer Folie, einer Membran, eines Sinterkörpers oder eines Schaums vorliegt.

3. Verfahren nach einem der vorhergehenden Ansprüche, **dadurch gekennzeichnet, dass** die funktionellen Gruppen direkt an das Trägermaterial gebunden sind.

4. Verfahren nach einem der vorhergehenden Ansprüche, **dadurch gekennzeichnet, dass** die funktionellen Gruppen über einen Spacer an das Trägermaterial gebunden sind.

5. Verfahren nach einem der vorhergehenden Ansprüche, **dadurch gekennzeichnet, dass** das funktionalisierte Trägermaterial neutralen, kationischen oder anionischen Charakter besitzt.

6. Verfahren nach einem der vorhergehenden Ansprüche, **dadurch gekennzeichnet, dass** die Zellen ausgewählt sind aus prokaryontischen und eukaryontischen Zellen sowie Archaebakterien.

7. Verfahren nach dem vorhergehenden Anspruch, **dadurch gekennzeichnet, dass** die Zellen ausgewählt sind aus der Gruppe bestehend aus Bakterien, Pilzen, Algen, Sporen, pflanzlichen Zellen, tierischen Zellen, Zellen aus Zellkulturen und gentechnologisch veränderten Zellen.

8. Verfahren nach einem der vorhergehenden Ansprüche, **dadurch gekennzeichnet, dass** das Trägermaterial in dem flüssigen Medium dispergiert wird.

9. Verfahren nach einem der vorhergehenden Ansprüche, **dadurch gekennzeichnet, dass** das Trägermaterial von dem flüssigen Medium umströmt wird.

10. Verfahren nach einem der vorhergehenden Ansprüche, **dadurch gekennzeichnet, dass** das Trägermaterial in Form eines porösen Körpers vorliegt und das flüssige Medium dessen Poren durchströmt.

11. Verfahren nach einem der vorhergehenden Ansprüche, **dadurch gekennzeichnet, dass** die Abtrennung des Trägermaterials vom flüssigen Medium mittels Filtration, Sedimentation oder Zentrifugation erfolgt.

12. Verwendung des Verfahrens nach einem der Ansprüche 1 bis 11 zur Abtrennung von Zellen aus Flüssigkeiten, insbesondere Wasser, flüssi-gen Lebensmitteln, Flüssigkeiten aus Lebensmitteln, Körperflüssigkeiten oder Ausscheidungen.

## Claims

1. Method for the non-specific separation and analysis of cells from liquid media, in which method
a) a carrier material, selected from the group consisting of polymers, ceramics and glasses, is activated, the activation being effected by an oxidative activation with an oxidant, by a photochemical activation or by plasma treatment, and is then chemically functionalised in a hydrophilic manner on the surface in order to enable a chemisorption of the cells, the chemisorption being based on an ionic interaction and the functional groups being selected from the group consisting of ammonium, carboxyl, carboxylate, sulphonic acid, sulphonate and phosphate groups, and/or being based on covalent interactions, the functional groups being selected from the group consisting of active esters, acid amides, halides and acid halides,
b) the carrier material is brought in contact with the liquid medium and the cells are adsorbed on the carrier material,
c) the carrier material laden with the cells is separated from the liquid medium, and
d) at least some of the cells are recovered by elution of the laden carrier material with a liquid and the recovered cells are analysed for qualitative determination.

2. Method according to the preceding claim, **characterised in that** the carrier material is present in the form of a powder, a granulate, a film, a membrane, a sintered body or a foam.

3. Method according to one of the preceding claims, **characterised in that** the functional groups are bonded directly to the carrier material.

4. Method according to one of the preceding claims, **characterised in that** the functional groups are bonded to the carrier material via a spacer.

5. Method according to one of the preceding claims, **characterised in that** the functionalised carrier material has a neutral, cationic or anionic character.

6. Method according to one of the preceding claims, **characterised in that** the cells are selected from prokaryotic and eukaryotic cells and archaebacteria.

7. Method according to the preceding claim, **characterised in that** the cells are selected from the group consisting of bacteria, fungi, algae, spores, plant cells, animal cells, cells from cell cultures and genetically modified cells.

8. Method according to one of the preceding claims, **characterised in that** the carrier material is dispersed in the liquid medium.

9. Method according to one of the preceding claims, **characterised in that** the carrier material is subjected to a flow of the liquid medium.

10. Method according to one of the preceding claims, **characterised in that** the carrier material is present in the form of a porous body and the liquid medium flows through the pores thereof.

11. Method according to one of the preceding claims, **characterised in that** the separation of the carrier material from the liquid medium is effected by means of filtration, sedimentation or centrifugation.

12. Use of the method according to one of claims 1 to 11 for the separation of cells from liquids, in particular water, liquid foods, liquids from foods, body fluids or excretions.

## Revendications

1. Procédé pour la séparation non spécifique et l'analyse de cellules contenues dans des milieux liquides, selon lequel
a) on active un matériau support, choisi parmi le groupe consistant en des polymères, des céramiques et des verres, l'activation étant réalisée par une activation oxydative à l'aide d'un oxydant, par une activation photochimique ou par un traitement par plasma, puis on procède à une fonctionnalisation chimique hydrophile de sa surface pour permettre une chimisorption des cellules, la chimisorption reposant sur une interaction ionique et les groupes fonctionnels étant choisis parmi le groupe consistant en des groupes ammonium, carboxyle, carboxylate, acide sulfonique, sulfate et phosphate et/ou sur des interactions covalentes, les groupes fonctionnels étant choisis parmi le groupe consistant en des esters actifs, des amides d'acides, des halogénures et des halogénures d'acyle,
b) on met le matériau support en contact avec le milieu liquide et on fait adsorber les cellules par le matériau support,
c) on sépare le matériau support, chargé en cellules, du milieu liquide et
d) on récupère au moins une partie des cellules par élution, à l'aide d'un liquide, du matériau support chargé et on analyse les cellules récupérées pour une détermination qualitative.

2. Procédé selon la revendication précédente, **caractérisé en ce que** le matériau support se présente sous forme d'une poudre, d'un granulé, d'une feuille, d'une membrane, d'un corps fritté ou d'une mousse.

3. Procédé selon l'une des revendications précédentes, **caractérisé en ce que** les groupes fonctionnels sont directement liés au matériau support.

4. Procédé selon l'une des revendications précédentes, **caractérisé en ce que** les groupes fonctionnels sont liés au matériau support par l'intermédiaire d'un espaceur.

5. Procédé selon l'une des revendications précédentes, **caractérisé en ce que** le matériau support fonctionnalisé présente un caractère neutre, cationique ou anionique.

6. Procédé selon l'une des revendications précédentes, **caractérisé en ce que** les cellules sont choisies parmi des cellules procaryotes et des cellules eucaryotes, ainsi que des archaebactéries.

7. Procédé selon la revendication précédente, **caractérisé en ce que** les cellules sont choisies dans le groupe consistant en des bactéries, des champignons, des algues, des spores, des cellules végétales, des cellules animales, des cellules provenant de cultures cellulaires et des cellules génétiquement modifiées.

8. Procédé selon l'une des revendications précédentes, **caractérisé en ce qu'**on disperse le matériau support dans le milieu liquide.

9. Procédé selon l'une des revendications précédentes, **caractérisé en ce que** le milieu liquide s'écoule autour du matériau support.

10. Procédé selon l'une des revendications précédentes, **caractérisé en ce que** le matériau support se présente sous forme d'un corps poreux et **en ce que** le milieu liquide traverse ses pores.

11. Procédé selon l'une des revendications précédentes, **caractérisé en ce que** la séparation du matériau support du milieu liquide s'effectue par filtration, sédimentation ou centrifugation.

12. Utilisation du procédé selon l'une des revendications 1 à 11 pour séparer des cellules de liquides, en particulier de l'eau, des aliments liquides, des liquides de produits alimentaires, de liquides corporels ou d'excrétions.
